# EUROPEAN PATENT APPLICATION

(11) **EP 3 351 556 A1**
(43) Date of publication of application: **25.07.2018**
(21) Application number: 16846575.5
(22) Date of filing: 15.09.2016
(51) Int. Cl.: C07K 14/435, C12N 15/09, C12Q 1/70, G01N 33/53, G01N 33/566

(54) **PROTEIN TAG THAT BINDS TO SUBSTANCES HAVING A CRYSTAL STRUCTURE, AND USES THEREOF**

(30) Priority: 18.09.2015 JP 2015185940
(71) Applicant: Hiroshima University, Higashi-Hiroshima-shi Hiroshima 739-8511 (JP)
(72) Inventor: KURODA, Akio, Higashi-Hiroshima-shi Hiroshima 739-8530 (JP); ISHIDA, Takenori, Higashi-Hiroshima-shi Hiroshima 739-8530 (JP)
(74) Representative: Bittner, Thomas L.
(86) International application number: PCT/JP2016/077292
(87) International publication number: WO 2017/047704

(57) **Abstract**

In order to provide a protein tag and use thereof, the present invention uses a protein tag that binds to a substance having a crystal structure and that includes a binding domain whose amino acid sequence is a mutated version of an amino acid sequence of an ice-binding domain derived from an antifreeze protein.

## Description

The present invention refers to a protein tag that binds to substances having a crystal structure, and uses thereof.

### Background

The present invention relates to a protein tag which binds to a substance having a crystal structure, and use of the protein tag.

There are various substances in the environment surrounding humans, some are harmless to human bodies and some are harmful to human bodies. For example, asbestos has conventionally been used widely as a construction material. In recent years, increasing recognition of significance of the effect of asbestos on human bodies has led to a need for testing whether or not asbestos is used as a material for a building or the like. In addition to asbestos, there are also many substances harmful to human bodies in the environment surrounding humans, and there has been a need for testing whether or not such substances are used as materials for buildings or the like.

A technique conventionally used as a method of detecting asbestos is to confirm the presence of asbestos by: allowing a complex (a complex of (i) a binding protein that has the ability to bind to asbestos and (ii) a marker) to bind to asbestos; and detecting the marker bound to asbestos (for example, refer to Patent Literatures 1 to 3).

For example, JP 2009-31062 discloses a microorganism-derived protein as a binding protein and discloses alkaline phosphatase as a marker.

JP 2010-32271 discloses GatZ protein, L1 protein, L5 protein, S1 protein, S4 protein, and S7 protein as binding proteins, and discloses fluorescent substance, fluorescent protein, lucifer-ase, alkaline phosphatase, beta galactosidase, diaphorase, and peroxidase as markers.

WO 2013/024881 A1 discloses H-NS protein, DksA protein, and GatZ protein as binding proteins, and discloses Cy3, DyLight488, fluorescein, Alexa488, ATTO488, CF488A, DyLight550, and CF555 as markers.

### Summary

A to-be-detected substance such as asbestos is sometimes detected in bright light. What is important in this case is to use a binding protein that has a high ability to bind to the to-be-detected substance to thereby cause many markers (for example, markers detectable by fluorescence) to bind to the to-be-detected substance, thereby increasing the detectability of the markers.

An example of a place where asbestos (which is an example of the to-be-detected substance) is to be detected is a place where a building, which was built possibly using asbestos, is demolished. In this case, asbestos is to be detected outdoors, or indoors in bright light. This increases the necessity to use a binding protein that has a high ability to bind to asbestos to thereby cause many markers to bind to asbestos.

Although existing binding proteins do have a high ability to bind to a to-be-detected substance, under the above-mentioned circumstances, there has been a demand for finding a binding protein with a higher ability to bind to a to-be-detected substance.

The present invention was made in view of the foregoing issues, and an object thereof is to provide a protein tag and use of the protein tag.

Antifreeze proteins are proteins which specifically bind to surfaces of ice crystals forming in cells to thereby inhibit growth of the ice crystals, and which thereby prevent living organisms from freezing. Many antifreeze proteins have been identified hitherto, and ice-binding domains, which are involved in binding to the surfaces of ice crystals and which are present in these antifreeze proteins, have been identified hitherto. The ice-binding domains are known to have a characteristic planar structure, and specific amino acids in the planar structure have been found to be involved in binding to the surfaces of ice crystals (for example, refer to (i) Peter L. Davies, Ice-binding proteins: a remarkable diversity of structures for stopping and starting ice growth, Trends in Biochemical Science, November 2014, Vo. 39, No. 11, pp. 548-555, (ii) Zongchao Jia et. al., Antifreeze proteins: an unusual receptor-ligand interaction, Trends in Biochemical Science, February 2002, Vo. 27, No. 2, pp. 101-106, (iii) Shotaro TANAKA et. al., "Antifreeze Protein", SEIBUTSU BUTSURI 43(3), pp. 130-135 (2003), (iv) Yih-Cherng Liou et. al., Mimicry of ice structure by surface hydroxyls and water of a beta-helix antifreeze protein, Nature, Vol. 406, 20 July 2000, pp. 322-324).

The inventors studied hard in view of the foregoing object, and found that (i) binding domains whose amino acid sequences are mutated versions of an amino acid sequence of an ice-binding domain derived from an antifreeze protein (in other words, mutants of an ice-binding domain) are capable of binding to various substances having a crystal structure and that (ii) the type of substance to which a binding domain binds can be changed by changing the type of mutation. On the basis of these findings, the inventors accomplished the present invention.

In order to attain the foregoing object, a protein tag of the present invention is a protein tag which binds to a substance having a crystal structure, the protein tag including a binding domain whose amino acid sequence is a mutated version of an amino acid sequence of an ice-binding domain derived from an antifreeze protein.

In order to attain the foregoing object, a method for screening a protein tag which binds to a target substance having a crystal structure of the present invention includes the steps of: bringing a phage and a target substance having a crystal structure into contact with each other, the phage displaying, by a phage display method, a protein tag that includes a binding domain whose amino acid sequence is a mutated version of an amino acid sequence of an ice-binding domain derived from an antifreeze protein; and recovering the phage bound to the target substance having a crystal structure.

In order to attain the foregoing object, a kit for screening a protein tag which binds to a target substance having a crystal structure of the present invention includes a phage vector or a phagemid vector each of which contains a gene coding for a protein tag, the protein tag including a binding domain whose amino acid sequence is a mutated version of an amino acid sequence of an ice-binding domain derived from an antifreeze protein.

In order to attain the foregoing object, a method for producing a protein tag which binds to a target substance having a crystal structure of the present invention includes the steps of: bringing a phage and a target substance having a crystal structure into contact with each other, the phage displaying, by a phage display method, a protein tag that includes a binding domain whose amino acid sequence is a mutated version of an amino acid sequence of an ice-binding domain derived from an antifreeze protein; and recovering the phage bound to the target substance having a crystal structure.

The present invention provides the following effect: it is possible to realize a protein tag that has a high ability to bind to a substance having a crystal structure. The protein tag of the present invention provides, for example, the following effect: since a protein tag of the present invention has a high ability to bind a to-be-detected substance, in a case where a fluorescent substance is used as a marker, it is possible to detect the to-be-detected substance with high sensitivity even under a bright environment.

The present invention provides the following effect: it is possible to prepare a protein tag that is capable of binding to an intended substance, by changing the type of mutation that occurs in an amino acid sequence of an ice-binding domain derived from an antifreeze protein.

### Description of further embodiments

Following, embodiments, by way of example, are described with reference to figures. In the figures show:
- Fig. 1: illustrates binding between H-NS60-90 and asbestos in Examples of the present invention.
- Fig. 2: illustrates binding between an amino-acid-substituted TmAFP and asbestos, in Examples of the present invention.
- Fig. 3: illustrates binding between an amino-acid-substituted TmAFP and asbestos, in Examples of the present invention.
- Fig. 4: illustrates binding between an amino-acid-substituted TmAFP and asbestos, in Examples of the present invention.
- Fig. 5: illustrates binding between an amino-acid-substituted TmAFP and asbestos and binding between the amino-acid-substituted TmAFP and various JFM-standard fibrous samples, in Examples of the present invention.
- Fig. 6: illustrates binding between an amino-acid-substituted TmAFP and various JFM-standard fibrous samples, in Examples of the present invention.
- Fig. 7: illustrates binding between an amino-acid-substituted TmAFP and asbestos, in Examples of the present invention.
- Fig. 8: illustrates binding between an amino-acid-substituted TmAFP and titanium oxide, in Examples of the present invention.

An embodiment of the present invention is described below. Note, however, that the present invention is not limited to such an embodiment. The present invention is not limited to arrangements described below, but can be altered within the scope of the claims. An embodiment or example derived from a combination of technical means disclosed in different embodiments or examples is also encompassed in the technical scope of the present invention. All academic and patent literatures listed herein are incorporated herein by reference. Note that a numerical range "A to B" herein means "not less than A and not more than B" unless otherwise specified in this specification. Furthermore, in a case where an amino acid sequence is written as "-X1X2X3-" in this specification, the "X1" situated at the left indicates an amino acid at the amino-terminus, whereas the "X3" situated at the right indicates an amino acid at the carboxyl-terminus.

A protein tag of the present embodiment binds to a substance having a crystal structure. The protein tag includes a binding domain whose amino acid sequence is a mutated version of an amino acid sequence of an ice-binding domain derived from an antifreeze protein (in other words, the protein tag includes a mutant of an ice-binding domain).

As used herein, the term "crystal structure" means a state in which atoms, ions, or molecules are regularly arranged in three dimensions to form a space lattice. On the other hand, an ice-binding domain derived from an antifreeze protein has a characteristic planar structure, in which specific amino acids are arranged regularly with specific distances between them. The ice-binding domain is thus suitable as a molecular framework of a protein that binds to a substance having a crystal structure.

The protein tag of the present invention is such that, in its binding domain, specific amino acids (e.g., amino acids at which a mutation has occurred) are arranged in-plane with a certain distance between them. When this distance and the lattice spacing of the space lattice are substantially equal to each other, the protein tag of the present invention is capable of binding to the substance having a crystal structure in a suitable manner. The protein tag of the present invention can be configured such that specific amino acids have an intended distance between them, by appropriately selecting the type of mutation. Accordingly, the protein tag of the present invention is capable of binding to an intended substance that has a space lattice whose spacing is substantially equal to the foregoing distance.

As used herein, the term "protein tag" means a tag containing a polypeptide as a constituent or a tag consisting of a polypeptide. The protein tag of the present invention may be (1) a tag that individually binds to a substance having a crystal structure and thereafter is detected using a certain marker (in other words, a mark used to detect the substance having a crystal structure) or may be (2) linked with a marker in advance to form a complex. The complex is then allowed to bind to a substance having a crystal structure and thereafter the marker is detected.

A substance to which the protein tag of the present embodiment binds varies depending on the type of mutation that occurs in the amino acid sequence of the ice-binding domain derived from an antifreeze protein. Therefore, there is no limitation on a substance to which the protein tag binds.

Examples of a substance to which the protein tag of the present embodiment binds (in other words, a substance having a crystal structure) include asbestos (specifically, amosite, crocidolite, and chrysotile), titanium oxide, silicon carbide whisker, zinc oxide, gold, silver, platinum, quartz, iridium oxide, iron oxide, and hydroxyapatite. Note, however, that the substance in the present invention is not limited to the substances listed above, as explained earlier.

The protein tag of the present embodiment includes a binding domain whose amino acid sequence is a mutated version of an amino acid sequence of an ice-binding domain derived from an antifreeze protein.

Antifreeze proteins have ice-binding domains having similar structures. In the present invention, mutants of those ice-binding domains having similar structures can be used. Therefore, there is no particular limitation on the antifreeze protein from which the ice-binding domain is derived, and thus any antifreeze protein can be employed. The following table provides a list of example antifreeze proteins. Note, however, that these antifreeze proteins do not impose any limitation on the present invention.

**Table 1**

| No. | Type | Protein | Organism (origin) | Amino acid sequence and gene information | | Structure in-formation |
|---|---|---|---|---|---|---|
| | | | | Database | Accession No. | PDB No. |
| 1 | Fish | Maxi (Hyperactive Type I AFP) | Pseudopleuronectes americanus (Winter flounder) | GenBank | ABX38716 | 4KE2 |
| 2 | | Type I AFP | Pseudopleuronectes americanus (Winter flounder) | GenBank | AAB59964 | 1WFA |
| 3 | | Type II AFP | Clupea harengus (Atlantic herring) | GenBank | AAA49200 | 2PY2 |
| 4 | | Type II AFP | Hemitripterus americanus (Sea raven) | GenBank | AAA49618 | 2AFP |
| 5 | | Type II AFP | Brachyopsis rostratus (longsnout poacher) | GenBank | BAF37106 | 2ZIB |
| 6 | | Type II AFP | Osmerus mordax (rainbow smelt) | GenBank | M96154 | - |
| 7 | | Type III AFP | Macrozoarces americanus(Ocean pout) | PIR-PSD | A30839 | 1AME |
| 8 | | Type III AFP RD3 | Lycodichthys dearborni (Antarctic eelpout) | PIR-PSD | S53514 | 1C89 |
| 9 | | Type IV AFP LS-12 | Myoxocephalus octodecimspinosis (long-horn sculpin) | GenBank | AF026525 | - |
| 10 | | Type IV AFP | Pleuragramma antarcticum (Antarctic silverfish) | GenBank | HM800727 | - |
| 11 | | Type IV AFP | Notothenia coriiceps (black rockcod) | GenBank | HM800728 | - |
| 12 | Plant | Lolium perenne AFP (LpAFP) | Lolium perenne | GenBank | ACG63783 | 3ULT |
| 13 | Insect | Spruce budworm AFP (SbwAFP) | Choristoneura fumiferana (Spruce budworm) | GenBank | AAG32660 | 1EWW |
| 14 | | Choristoneura fumiferana AFP Isoform-501 (CfAFP-501) | Choristoneura fumiferana (Spruce budworm) | GenBank | AAF86611 | 1M8N |
| 15 | | Tenebrio molitor AFP (TmAFP) | Tenebrio molitor (yellow mealworm) | GenBank | AAB70750 | 1EZG |
| 16 | | Rhagium inquisitor AFP (RiAFP) | Rhagium inquisitor | GenBank | ADR80610 | 4DT5 |
| 17 | | Snow Flea AFP (SfAFP) | Hypogastrura harveyi (Snow Flea) | GenBank | ABB03725 | 2PNE |
| 18 | Microorganism | Typhula ishikariensis AFP (TisAFP) | Typhula ishikariensis | GenBank | BAD02893 | 3VN3 |
| 19 | | Marinomonas primoryensis AFP (MpAFP) | Marinomonas primoryensis | GenBank | ABL74378 | 3P4G |

The mutation that occurs in the amino acid sequence of the ice-binding domain derived from an antifreeze protein is not limited to a particular type, and may be amino acid substitution, amino acid deletion, or amino acid addition. Of these types of mutation listed above, amino acid substitution is preferred, in order to better maintain the structure (in other words, planar structure) of a wild-type ice-binding domain in the binding domain and to increase the ability of the binding domain to bind to a substance.

In regard to the above-described mutation, there is no particular limitation on the number of amino acids, at which a mutation occurs, of the amino acid sequence of the ice-binding domain. The number of the amino acids may be 1 or more, 2 or more, 3 or more, 4 or more, 5 or more, 6 or more, 7 or more, 8 or more, 9 or more, or 10 or more. In regard to the above-described mutation, there is no particular limitation on the upper limit of the number of amino acids, at which a mutation occurs, of the amino acid sequence of the ice-binding domain. The upper limit may be, for example, 20, 18, 16, 14, 12, or 10.

The mutation that occurs in the amino acid sequence of the ice-binding domain derived from an antifreeze protein is preferably amino acid substitution, which is such that threonine in the wild-type ice-binding domain is substituted with an amino acid "a" different from threonine. With this arrangement, the binding specificity of the protein tag (in other words, type of substance to which the protein tag binds, and/or, the binding force of the protein tag with respect to a specific substance) can be more efficiently changed, and the ability of the protein tag to bind to a substance can be more efficiently increased. In a case where a plurality of threonines are substituted, these threonines may be substituted with amino acids of one kind or may be substituted with amino acids of two or more kinds. In order to increase the binding specificity of the protein tag to a greater extent, it is preferable that the threonines are substituted with amino acids of one kind.

The amino acid "a" is not limited, provided that it is an amino acid different from threonine. Examples of the amino acid "a" include glycine, alanine, valine, leucine, isoleucine, tyrosine, cysteine, methionine, asparatic acid, asparagine, glutamic acid, glutamine, arginine, lysine, histidine, phenylalanine, tyrosine, tryptophane, and proline. The binding specificity of the protein tag can be changed by selecting an appropriate amino acid "a".

For example, when lysine is selected as the amino acid "a", it is possible to realize a protein tag that highly specifically binds to asbestos (e.g., amosite or crocidolite) or silicon carbide whisker. When glutamic acid is selected as the amino acid "a", it is possible to realize a protein tag that highly specifically binds to asbestos (e.g., chrysotile). When histidine is selected as the amino acid "a", it is possible to realize a protein tag that highly specifically binds to titanium oxide. It should be understood that the amino acid "a" in the present invention is not limited to these examples.

By preparing a protein tag library that has various amino acids each serving as an amino acid "a", bringing the library into contact with an intended substance, and identifying a protein tag bound to the intended substance (in other words, identifying the type of amino acid "a" of the protein tag bound to the substance), it is possible to find a protein tag that is capable of binding to the intended substance. It is clear also from this that there is no limitation on the amino acid "a" in the present invention. It will be further understood from the [Screening method] section (described later) and the "Screening kit" section (described later) that there is no limitation on the amino acid "a" in the present invention.

The protein tag of the present embodiment may be configured such that: the ice-binding domain of a wild type contains a motif represented by the following amino acids; and the threonine substituted is at least one selected from the group consisting of threonine at the 1st position from the amino-terminus of the motif and threonine at the 3rd position from the amino-terminus of the motif:
Motif: -TCTXSXXCXXAX- ... (SEQ ID NO:1)
(where T represents threonine, C represents cysteine, S represents serine, A represents alanine, and X represents any amino acid.)

The ice-binding domain of a wild type may be constituted by one such motif or may be constituted by a plurality of such motifs (e.g., 2 or more, 3 or more, 4 or more, or 5 or more motifs). The ice-binding domain of a wild type may be a domain containing such a motif(s) or may be a domain consisting of such a motif(s).

The term "domain containing such a motif(s)" means a domain in which, assuming that the total number of amino acids constituting the ice-binding domain is x and the total number of amino acids constituting the motif(s) contained in the ice-binding domain is y, the value of "y/x×100" is 30% or greater, more preferably 40% or greater, still more preferably 50% or greater, still more preferably 60(%) or greater, still more preferably 70(%) or greater, still more preferably 80(%) or greater, still more preferably 90(%) or greater, still more preferably 95(%) or greater, most preferably 98(%) or greater. The upper limit of the value of "y/x×100" is not particularly limited, and can be, for example, 98(%) or 100(%).

More specifically, the ice-binding domain of a wild type may be a domain that contains a polypeptide constituted by the following amino acid sequence of SEQ ID NO:2 or SEQ ID NO:3, or may be a domain consisting of a polypeptide constituted by the following amino acid sequence of SEQ ID NO:2 or SEQ ID NO:3.
- ANTCTGSTDCNTAQTCTNSKDCFEA- (SEQ ID NO:2)
- NAVTCTNSQHCVKANTCTGSTDCNTAQTCTNSKDCFEANTCTDSTNCY-(SEQ ID NO:3)

One known example of an antifreeze protein is TmAFP (base sequence: SEQ ID NO:4, amino acid sequence: SEQ ID NO:5). TmAFP is formed in the following manner: a protein is first produced in accordance with the base sequence of SEQ ID NO:4 and then a signal sequence, which corresponds to the amino acids at the 1st to 28th positions from the amino-terminus of the protein, is cleaved and removed, whereby a mature TmAFP is formed.

A polypeptide constituted by the amino acid sequence of SEQ ID NO:2 is a region consisting of the amino acids at the 37th to 61st positions from the amino-terminus of a mature TmAFP (with methionine added at the amino-terminus), whereas a polypeptide constituted by the amino acid sequence of SEQ ID NO:3 is a region consisting of the amino acids at the 24th to 71st positions from the amino-terminus of the mature TmAFP.

In the case of the foregoing amino acid sequence of SEQ ID NO:2, in a mutant of the ice-binding domain, at least one of threonine at the 3rd position, threonine at the 5th position, threonine at the 15th position, and threonine at the 17th position, from the amino-terminus of the amino acid sequence of SEQ ID NO:2, may be substituted, for example.

In the case of the foregoing amino acid sequence of SEQ ID NO:3, in a mutant of the ice-binding domain, at least one of threonine at the 16th position, threonine at the 18th position, threonine at the 28th position, and threonine at the 30th position, from the amino-terminus of the amino acid sequence of SEQ ID NO:3, may be substituted, for example.

In a case where a wild-type ice-binding domain contains an amino acid of the same kind as the amino acid "a", a mutant of the ice-binding domain is preferably such that the amino acid, which is originally contained in the wild-type ice-binding domain, of the same kind as the amino acid "a" is substituted with an amino acid "b" different from the amino acid "a".

As discussed earlier, the mutation that occurs in the amino acid sequence of the ice-binding domain derived from an antifreeze protein is preferably amino acid substitution, which is such that threonine in the ice-binding domain of a wild type is substituted with an amino acid "a" different from threonine. In this case, the amino acid "a", which is a substitute for threonine, plays an important role in binding of the protein tag to a substance. If an undesired amino acid "a" were present in the mutant of the ice-binding domain, this undesired amino acid "a" would influence the binding specificity of the protein tag. In this regard, there is no undesired amino acid "a" in the mutant of the ice-binding domain of the above arrangement. Therefore, the amino acid "a", which plays an important role, works normally and makes it possible to increase the binding specificity of the protein tag.

The amino acid "b" is not limited, provided that it is an amino acid of a different kind from the amino acid "a". Examples of the amino acid "b" include glycine, alanine, valine, leucine, isoleucine, tyrosine, cysteine, methionine, asparatic acid, asparagine, glutamic acid, glutamine, arginine, lysine, histidine, phenylalanine, tyrosine, tryptophane, and proline.

The amino acid "b" is preferably an amino acid that little influences the structure of the protein (e.g., glycine or alanine), from the viewpoint that such an amino acid does not impose undesired effects on the binding specificity of the protein tag, in other words, from the viewpoint that the binding specificity of the protein tag is increased.

The protein tag of the present embodiment can also be configured as described below.

In the protein tag of the present embodiment, mutation in the mutated version is preferably amino acid substitution.

In the protein tag of the present embodiment, the mutation is preferably such that threonine in the ice-binding domain of a wild type is substituted with an amino acid "a" different from threonine.

It is preferable in the protein tag of the present embodiment that: the ice-binding domain of a wild type contains a motif represented by the following amino acids; and the threonine substituted is at least one selected from the group consisting of threonine at the 1st position from the amino-terminus of the motif and threonine at the 3rd position from the amino-terminus of the motif:
Motif: -TCTXSXXCXXAX-
(where T represents threonine, C represents cysteine, S represents serine, A represents alanine, and X represents any amino acid).

It is preferable in the protein tag of the present embodiment that, in a case where the ice-binding domain of a wild type contains an amino acid of the same kind as the amino acid "a", the amino acid, which is originally contained, of the same kind as the amino acid "a" is substituted with an amino acid "b" different from the amino acid "a".

### [2. Method for screening protein tag which binds to target substance having crystal structure]

A substance to which the foregoing protein tag binds varies depending on the type of mutation that occurs in an amino acid sequence of an ice-binding domain derived from an antifreeze protein. This indicates that there is a possibility that various protein tags having various mutations (in other words, protein tag library) include a protein tag that is capable of binding to an intended substance.

In view of this, a method for screening a protein tag which binds to a target substance having a crystal structure of the present embodiment includes the steps of: bringing a phage (in other words, a phage library) and a target substance having a crystal structure into contact with each other, the phage displaying, by a phage display method, a protein tag that includes a binding domain whose amino acid sequence is a mutated version of an amino acid sequence of an ice-binding domain derived from an antifreeze protein; and recovering the phage bound to the target substance having a crystal structure.

According to the screening method of the present embodiment, the step of bringing a phage and a target substance having a crystal structure into contact with each other allows the phage, which displays a specific protein tag, to specifically bind to the target substance, and the step of recovering the phage involves selectively recovering only the phage specifically bound to the target substance. By identifying the protein tag displayed on the recovered phage (in other words, by reading an amino acid sequence of the protein tag displayed on the recovered phage or reading a base sequence of the protein tag displayed on the recovered phage), it is possible to identify a protein tag which specifically binds to the target substance.

The phage display method itself is an already established technique. Therefore, the screening method of the present embodiment can be carried out basically in accordance with a well-known phage display method.

The phage is preferably a phage library, which is a population of phages displaying various protein tags having various mutations. With this configuration, it is possible to efficiently obtain a protein tag that is capable of binding to an intended substance. Since the details of the protein tag have already been described, the description for the protein tag is omitted here.

There is no particular limitation on vectors for use in the phage display method. The vectors can be phage vectors and/or phagemid vectors. More specifically, the vectors can be filamentous fd phage (M13 phage), λ phage, T4 phage, and/or T7 phage. It should be understood that the present invention is not limited by those listed above.

To a vector described above, a gene coding for a protein tag is inserted. The protein tag includes a binding domain whose amino acid sequence is a mutated version of an amino acid sequence of an ice-binding domain derived from an antifreeze protein. Since the insertion of the gene into the vector can be carried out in accordance with a well-known gene engineering technique, the description of how to insert the gene is omitted here.

The screening method of the present embodiment can also be arranged as described below. It should be understood that the present invention is not limited to those described below.

In the screening method of the present embodiment, mutation in the mutated version is preferably amino acid substitution.

In the screening method of the present embodiment, the mutation is preferably such that threonine in the ice-binding domain of a wild type is substituted with an amino acid "a" different from threonine.

It is preferable in the screening method of the present embodiment that: the ice-binding domain of a wild type contains a motif represented by the following amino acids; and the threonine substituted is at least one selected from the group consisting of threonine at the 1st position from the amino-terminus of the motif and threonine at the 3rd position from the amino-terminus of the motif:
Motif: -TCTXSXXCXXAX-
(where T represents threonine, C represents cysteine, S represents serine, A represents alanine, and X represents any amino acid).

It is preferable in the screening method of the present embodiment that, in a case where the ice-binding domain of a wild type contains an amino acid of the same kind as the amino acid "a", the amino acid, which is originally contained, of the same kind as the amino acid "a" is substituted with an amino acid "b" different from the amino acid "a".

### [3. Kit for screening protein tag which binds to target substance having crystal structure]

A kit for screening a protein tag which binds to a target substance having a crystal structure of the present embodiment is a kit that can be used in carrying out the foregoing method for screening a protein tag which binds to a target substance having a crystal structure.

Specifically, a kit for screening a protein tag which binds to a target substance having a crystal structure of the present embodiment includes a phage vector or a phagemid vector each of which contains a gene coding for a protein tag, the protein tag including a binding domain whose amino acid sequence is a mutated version of an amino acid sequence of an ice-binding domain derived from an antifreeze protein.

Since the details of the protein tag have been already described, the description for the protein tag is omitted here. The gene coding for the protein tag is not limited, provided that the gene codes for the protein tag. There is no particular limitation on codons corresponding to the respective amino acids constituting the protein tag.

There is no particular limitation on the vector. The vector can be a phage vector or a phagemid vector. More specifically, the vector can be filamentous fd phage (M13 phage), λ phage, T4 phage, or T7 phage. It should be understood that the present invention is not limited by those listed above.

To the vector described above, a gene coding for a protein tag is inserted. The protein tag includes a binding domain whose amino acid sequence is a mutated version of an amino acid sequence of an ice-binding domain derived from an antifreeze protein. Since the insertion of the gene into the vector can be carried out in accordance with a well-known gene engineering technique, the description of how to insert the gene is omitted here.

The screening kit of the present embodiment can also be configured as described below. It should be understood that the present invention is not limited to those described below.

In the screening kit of the present embodiment, mutation in the mutated version is preferably amino acid substitution.

In the screening kit of the present embodiment, the mutation is preferably such that threonine in the ice-binding domain of a wild type is substituted with an amino acid "a" different from threonine.

It is preferable in the screening kit of the present embodiment that: the ice-binding domain of a wild type contains a motif represented by the following amino acids; and the threonine substituted is at least one selected from the group consisting of threonine at the 1st position from the amino-terminus of the motif and threonine at the 3rd position from the amino-terminus of the motif:
Motif: -TCTXSXXCXXAX-
(where T represents threonine, C represents cysteine, S represents serine, A represents alanine, and X represents any amino acid).

It is preferable in the screening kit of the present embodiment that, in a case where the ice-binding domain of a wild type contains an amino acid of the same kind as the amino acid "a", the amino acid, which is originally contained, of the same kind as the amino acid "a" is substituted with an amino acid "b" different from the amino acid "a".

### [4. Method for producing protein tag which binds to target substance having crystal structure]

A method for producing a protein tag which binds to a target substance having a crystal structure of the present embodiment includes the steps of: bringing a phage (in other words, a phage library) and a target substance having a crystal structure into contact with each other, the phage displaying, by a phage display method, a protein tag that includes a binding domain whose amino acid sequence is a mutated version of an amino acid sequence of an ice-binding domain derived from an antifreeze protein; and recovering the phage bound to the target substance having a crystal structure.

According to the production method of the present embodiment, the step of bringing a phage and a target substance having a crystal structure into contact with each other allows the phage, which displays a specific protein tag, to specifically bind to the target substance, and the step of recovering the phage involves selectively recovering only the phage specifically bound to the target substance. By identifying the protein tag displayed on the recovered phage (in other words, by reading an amino acid sequence of the protein tag displayed on the recovered phage, or reading a base sequence of the protein tag displayed on the recovered phage), it is possible to identify a protein tag which specifically binds to the target substance.

The phage display method itself is an already established technique. Therefore, the production method of the present embodiment can be carried out basically in accordance with a well-known phage display method.

The phage is preferably a phage library, which is a population of phages displaying various protein tags having various mutations. With this configuration, it is possible to efficiently obtain a protein tag that is capable of binding to an intended substance. Since the details of the protein tag have already been described, the description for the protein tag is omitted here

There is no particular limitation on vectors for use in the phage display method. The vectors can be phage vectors and/or phagemid vectors. More specifically, the vectors can be filamentous fd phage (M13 phage), λ phage, T4 phage, and/or T7 phage. It should be understood that the present invention is not limited by those listed above.

To a vector described above, a gene coding for a protein tag is inserted. The protein tag includes a binding domain whose amino acid sequence is a mutated version of an amino acid sequence of an ice-binding domain derived from an antifreeze protein. Since the insertion of the gene into the vector can be carried out in accordance with a well-known gene engineering technique, the description of how to insert the gene is omitted here.

The production method of the present embodiment can also be arranged as described below. It should be understood that the present invention is not limited to those described below.

In the production method of the present embodiment, mutation in the mutated version is preferably amino acid substitution.

In the production method of the present embodiment, the mutation is preferably such that threonine in the ice-binding domain of a wild type is substituted with an amino acid "a" different from threonine.

It is preferable in the production method of the present embodiment that: the ice-binding domain of a wild type contains a motif represented by the following amino acids; and the threonine substituted is at least one selected from the group consisting of threonine at the 1st position from the amino-terminus of the motif and threonine at the 3rd position from the amino-terminus of the motif:
Motif: -TCTXSXXCXXAX-
(where T represents threonine, C represents cysteine, S represents serine, A represents alanine, and X represents any amino acid).

It is preferable in the production method of the present embodiment that, in a case where the ice-binding domain of a wild type contains an amino acid of the same kind as the amino acid "a", the amino acid, which is originally contained, of the same kind as the amino acid "a" is substituted with an amino acid "b" different from the amino acid "a".

The production method of the present embodiment may include, after the step of recovering the phage bound to the target substance having a crystal structure, a step of reading (i) an amino acid sequence of the protein tag displayed by the phage and/or (ii) a base sequence of the protein tag displayed by the phage. With this arrangement, it is possible to more easily produce the protein tag by a method of chemical polypeptide synthesis or a method of expressing a modified protein in a cell.

The production method of the present embodiment may include, after the step of reading (i) an amino acid sequence of the protein tag displayed by the phage and/or (ii) a base sequence of the protein tag displayed by the phage, a step of preparing the protein tag on the basis of the obtained amino sequence of the protein tag and/or a step of preparing a polynucleotide coding for the protein tag on the basis of the obtained base sequence of the protein tag. With this arrangement, it is possible to more easily produce the protein tag by a method of chemical polypeptide synthesis or a method of expressing a modified protein in a cell.

### Examples

### [1. Construction of protein expression vector]

### [1-1. Construction of biotinylated-protein-expression vector]

For preparation of a biotin-modified protein, a biotinylated-protein-expression vector was first constructed.

Inverse PCR was carried out using oligonucleotide primer P1 and oligonucleotide primer P2 and using, as a template, pET21-b (Novagen) which is a protein expression vector, and thereby a biotinylation Tag for biotinylation of a target protein was inserted upstream of HisTag in pET21-b. The specific base sequences of oligonucleotide primer P1 and oligonucleotide primer P2 are as follows.
P1:
   5'-GCTCAGAAAATCGAATGGCACGAACACCACCACCACCACCACTGAACTA-3' (SEQ ID NO:11)
P2:
   5'-CTCGAAGATGTCGTTCAGACCGCCACCCTCGAGTGCGGCCGCAAGCTTGTC-3' (SEQ ID NO:12)

The inverse PCR reaction was carried out with use of a KOD-plus-Mutagenesis Kit (TOYOBO) in accordance with the protocol of TOYOBO. The expression vector for expression of a biotinylated protein was named "pET21-AviTag-C".

### [1-2. Construction of expression vector for expression of H-NS60-90 (asbestos binding protein)]

PCR was carried out using oligonucleotide primer P3 and oligonucleotide primer P4 and using, as a template, a genome DNA of Escherichia coli K12, ATCC 700926, and thereby a gene coding for the amino acids at the 60th to 90th positions from the amino-terminus of H-NS (asbestos binding protein, Database: GenBank, Accession No.: AAC74319) was amplified. The specific base sequences of oligonucleotide primer P3 and oligonucleotide primer P4 are as follows.
P3:
   5'-CATATGCAATATCGCGAAATGCTGATC-3' (SEQ ID NO:13)
P4:
   5'-GGATCCAAACAACGTTTAGCTTTGGTGCC-3' (SEQ ID NO:14)

The PCR reaction was carried out with use of a KOD-plus Neo DNA polymerase (TOYOBO) in accordance with the protocol of TOYOBO.

The PCR amplified product and pET21-AviTag-C were each treated with the restriction enzymes Ndel and BamHI at 37°C for 1 hour, and then subjected to agarose gel electrophoresis. The amplified product and pET21-AviTag-C, which had been separated through agarose gel electrophoresis, were each cut from the agarose gel, the cut-out amplified product and pET21-AviTag-C were mixed together, and then ligated with use of a Ligation High (TOYOBO) at 16°C for 30 minutes.

Escherichia coli JM109 was transformed with the ligated product. From the thus-obtained transformant, pET21-AviTag-C to which the amplified product had been inserted was extracted, and this was named "pET-H-NS60-90-AviTag-C".

### [1-3. Construction of expression vector for expression of modified TmAFP]

An antifreeze protein from Tenebiro molitor (Tenebiro molitor Antifreeze protein: TmAFP, Database: GenBank, Accession No.: AAB70750) was used as an antifreeze protein.

In regard to the amino acid region (SEQ ID NO:2) at the 37th to 61st positions from the amino-terminus of TmAFP, threonines (T) at the 39th, 41st, 51st, and 53rd positions from the amino-terminus of TmAFP are believed to be involved in binding to an ice crystal. In consideration of this, a plurality of expression vectors for expression of modified TmAFPs, in each of which the threonines (T) at the foregoing four positions are substituted with some other amino acids in the amino acid region at the 37th to 61st positions of TmAFP, were first constructed.

### (Expression vector construction 1)

The following protein (T39K/T41K/T51K/T53K (SEQ ID NO:6)) was named "TmAFP37-61-KKKK": a protein having the same amino acid sequence as TmAFP except that, in regard to the amino acid region at the 37th to 61st positions from the amino-terminus of TmAFP, four threonines (T) at the 39th, 41st, 51st, and 53rd positions from the amino-terminus of TmAFP are each substituted with lysine (K).

For the construction of an expression vector for expression of the above protein, inverse PCR was carried out using oligonucleotide primer P5 and oligonucleotide primer P6 and using pET21-AviTag-C as a template, and thereby a gene coding for TmAFP37-61-KKKK was inserted upstream of a biotinylation Tag in pET21-AviTag-C. The specific base sequences of oligonucleotide primer P5 and oligonucleotide primer P6 are as follows.
P5:
   5'-AATAGCAAAGATTGTTTTGAAGCCGGTGGTGGTGGTAGCGGTGGCGGTCTGAACG ACATCTTCG-3' (SEQ ID NO:15)
P6:
   5'-TTTACATTTCTGTGCGGTATTACAATCGGTGCTACCTTTACATTTATTTGCCATATGT ATATCTCCTTCTTAAAG-3' (SEQ ID NO:16)

The inverse PCR reaction was carried out with use of a KOD-plus-Mutagenesis Kit (TOYOBO) in accordance with the protocol of TOYOBO. The expression vector for expression of this lysine-substituted TmAFP was named "pET21-TmAFP37-61-KKKK-AviTag-C".

### (Expression vector construction 2)

The following protein (T39K/T41K/T51K (SEQ ID NO:7)) was named "TmAFP37-61-KKK": a protein having the same amino acid sequence as TmAFP except that, in regard to the amino acid region at the 37th to 61st positions from the amino-terminus of TmAFP, only three threonines (T) at the 39th, 41st, and 51st positions, of the four threonines (T) at the 39th, 41st, 51st, and the 53rd positions from the amino-terminus of TmAFP, are each substituted with lysine (K).

For the construction of an expression vector for expression of the above protein, inverse PCR was carried out using oligonucleotide primer P5 and oligonucleotide primer P7 and using pET21-AviTag-C as a template, and thereby a gene coding for TmAFP37-61-KKK was inserted upstream of a biotinylation Tag in pET21-AviTag-C. The specific base sequences of oligonucleotide primer P5 and oligonucleotide primer P7 are as follows.
P5:
   5'-AATAGCAAAGATTGTTTTGAAGCCGGTGGTGGTGGTAGCGGTGGCGGTCTGAACG ACATCTTCG-3' (SEQ ID NO:15)
P7:
   5'-GGTACATTTCTGTGCGGTATTACAATCGGTGCTACCTTTACATTTATTTGCCATATGT ATATCTCCTTCTTAAAG-3' (SEQ ID NO:17)

The inverse PCR reaction was carried out with use of a KOD-plus-Mutagenesis Kit (TOYOBO) in accordance with the protocol of TOYOBO. The expression vector for expression of this lysine-substituted TmAFP was named "pET21-TmAFP37-61-KKK-AviTag-C".

### (Expression vector construction 3)

The following protein (T39E/T41E/T51E/T53E (SEQ ID NO:8)) was named "TmAFP37-61-EEEE": a protein having the same amino acid sequence as TmAFP except that, in regard to the amino acid region at the 37th to 61st positions from the amino-terminus of TmAFP, four threonines (T) at the 39th, 41st, 51st, and 53rd positions from the amino-terminus of TmAFP are each substituted with glutamic acid (E).

For the construction of an expression vector for expression of the above protein, inverse PCR was carried out using oligonucleotide primer P5 and oligonucleotide primer P8 and using pET21-AviTag-C as a template, and thereby a gene coding for TmAFP37-61-EEEE was inserted upstream of a biotinylation Tag in pET21-AviTag-C. The specific base sequences of oligonucleotide primer P5 and oligonucleotide primer P8 are as follows.
P5:
   5'-AATAGCAAAGATTGTTTTGAAGCCGGTGGTGGTGGTAGCGGTGGCGGTCTGAACG ACATCTTCG-3' (SEQ ID NO:15)
P8:
   5'-TTCACATTCCTGTGCGGTATTACAATCGGTGCTACCTTCACATTCATTTGCCATATGT ATATCTCCTTCTTAAAG-3' (SEQ ID NO:18)

The inverse PCR reaction was carried out with use of a KOD-plus-Mutagenesis Kit (TOYOBO) in accordance with the protocol of TOYOBO. The expression vector for expression of this glutamic-acid-substituted TmAFP was named "pET21-TmAFP37-61-EEEE-AviTag-C".

### (Expression vector construction 4)

The following protein (T39H/T41H/T51H/T53H (SEQ ID NO:9)) was named "TmAFP37-61-HHHH": a protein having the same amino acid sequence as TmAFP except that, in regard to the amino acid region at the 37th to 61st positions from the amino-terminus of TmAFP, four threonines (T) at the 39th, 41st, 51st, and 53rd positions from the amino-terminus of TmAFP are each substituted with histidine (H).

For the construction of an expression vector for expression of the above protein, inverse PCR was carried out using oligonucleotide primer P5 and oligonucleotide primer P9 and using pET21-AviTag-C as a template, and thereby a gene coding for TmAFP37-61-HHHH was inserted upstream of a biotinylation Tag in pET21-AviTag-C. The specific base sequences of oligonucleotide primer P5 and oligonucleotide primer P9 are as follows.
P5:
   5'-AATAGCAAAGATTGTTTTGAAGCCGGTGGTGGTGGTAGCGGTGGCGGTCTGAACG ACATCTTCG-3' (SEQ ID NO:15)
P9:
   5'-GTGACAGTGCTGTGCGGTATTACAATCGGTGCTACCGTGACAGTGATTTGCCATAT GTATATCTCCTTCTTAAAG-3' (SEQ ID NO:19)

The inverse PCR reaction was carried out with use of a KOD-plus-Mutagenesis Kit (TOYOBO) in accordance with the protocol of TOYOBO. The expression vector for expression of this histidine-substituted TmAFP was named "pET21-TmAFP37-61-HHHH-AviTag-C".

### (Expression vector construction 5)

In regard to the amino acid region (SEQ ID NO:3) at the 24th to 71st positions from the amino-terminus of TmAFP, threonines (T) at the 27th, 29th, 39th, 41st, 51st, 53rd, 63rd, and 65th positions from the amino-terminus of TmAFP are believed to be involved in binding to an ice crystal.

In consideration of this, the following protein (T39K/T41K/T51K/T53K/K36G/K56G (SEQ ID NO:10)) was named "TmAFP24-71-KKKK": a protein having the same amino acid sequence as TmAFP except that, in regard to the amino acid region at the 24th to 71st positions from the amino-terminus of TmAFP, threonines (T) at the 39th, 41st, 51st, and 53rd positions from the amino-terminus of TmAFP are each substituted with lysine (K), and lysines (K) at the 36th and 56th positions from the amino-terminus of TmAFP are each substituted with glycine (G).

An expression vector for expression of the above protein was constructed. First, a gene segment, which is such that NdeI and NotI restriction enzyme sites are introduced on the ends of a gene coding for the foregoing protein, was designed. This gene segment was synthesized by using an artificial gene synthesis service (GeneArt Strings DNA Fragments, LIFE TECHNOLOGY INC.) The specific base sequence of the gene segment is as follows.

5'-
GGAATTCCATATGAATGCAGTGACCTGTACCAATAGCCAGCATTGTGTTGGTGCCA
ATAAATGTAAAGGTAGCAC
CGATTGTAATACCGCACAGAAATGTAAAAATAGCGGTGATTGCTTTGAAGCCAATAC
CTGCACCGATAGCACCAATTGTTATGGTAGTGCGGCCGCAAGAGC-3' (SEQ ID
NO:20)

The foregoing gene segment and pET21-AviTag-C were treated with the restriction enzymes Ndel and Notl at 37°C for 1 hour, and then subjected to agarose gel electrophoresis. The gene segment and pET21-AviTag-C, which had been separated through agarose gel electrophoresis, were each cut from the agarose gel, the cut-out gene segment and pET21-AviTag-C were mixed together, and then ligated with use of a Ligation High (TOYOBO) at 16°C for 30 minutes.

Escherichia coli DH5a was transformed with the ligated product. From the thus-obtained transformant, pET21-AviTag-C to which the gene segment had been inserted was extracted, and this was named "pET21-TmAFP24-71-KKKK-AviTag-C".

### [2. Expression of proteins in Escherichia coli, and purification of proteins]

### [2-1. Expression and purification of asbestos-binding protein H-NS60-90]

For the expression of biotinylated H-NS60-90, BL21 (DE3) pBirAcm was used as a host. BL21 (DE3) pBirAcm is Escherichia coli BL21 (DE3) (Novagen) in which the biotin ligase expression vector pBirAcm is introduced.

Escherichia coli BL21 (DE3) pBirAcm, which had been transformed with a pET-H-NS60-90-AviTag-C plasmid, was cultured overnight at 37°C in a 2×YT medium containing carbenicillin (final concentration 50 mg/L) and chloramphenicol (30 mg/L).

The culture solution of Escherichia coli BL21 (DE3) pBirAcm was 1% (v/v) inoculated to a medium obtained by adding carbenicillin (final concentration 50 mg/L) and chloramphenicol (30 mg/L) to a 200 mL of a TYH medium [tryptone : 20 g/L, yeast extract: 10 g/L, HEPES: 11 g/L, NaCl: 5 g/L, magnesium sulfate: 1 g/L, glucose: 0.5%, pH has been adjusted to 7.2 to 7.4 by using potassium hydroxide].

Culture was carried out at 37°C until OD600 reached 0.6, and then IPTG (isopropyl-p-D-thioglactopyranoside) (final concentration 0.5 mM) and D-biotin (final concentration 100 µM) were added to the medium, and culture was carried out at 28°C for 4 hours.

Then, the culture solution was subjected to centrifugation, and thereby Escherichia coli BL21 (DE3) pBirAcm was recovered. To the thus-obtained Escherichia coli BL21 (DE3) pBirAcm, 10 mL of buffer A1 [0.05 M Tris-HCl (pH8.3), 0.05 M NaCl, 10% glycerol] was added to obtain a suspension of Escherichia coli BL21 (DE3) pBirAcm, and then Escherichia coli BL21 (DE3) pBirAcm was disrupted by ultrasonication.

The obtained disrupted cell solution was subjected to a Histrap FF column (GE Healthcare Bioscience), whereby biotinylated H-NS60-90 having histidine at the carboxyl-terminus was adsorbed to the column, and then the biotinylated H-NS60-90 was eluted from the column using buffer B1 [0.05 M Tris-HCl (pH 8.3), 0.05 M NaCl, 10% glycerol, 0.5 M imidazole].

The biotinylated H-NS60-90 thus obtained was checked for purification degree through polyacrylamide electrophoresis, and was found to have a purification degree of 95% or greater.

### [2-2. Expression of modified TmAFPs in Escherichia coli and purification of modified TmAFPs]

The following describes expression of TmAFP37-61-KKKK in Escherichia coli and a method for purifying TmAFP37-61-KKKK. Note that the expression of other modified TmAFPs in Escherichia coli and methods of purifying them are the same as those described below.

For the expression of biotinylated modified TmAFPs, SHuffle T7 pBirAcm was used as a host. SHuffle T7 pBirAcm is Escherichia coli SHuffle T7 Express (New England BioLabs) in which the biotin ligase expression vector pBirAcm is introduced.

Escherichia coli SHuffle T7 pBirAcm, which had been transformed with pET21-TmAFP37-61-KKKK-AviTag-C, was cultured overnight at 37°C in a TYH medium containing carbenicillin (final concentration 50 mg/L) and chloramphenicol (30 mg/L).

The culture solution of Escherichia coli SHuffle T7 pBirAcm was 2.5% (v/v) inoculated to a medium obtained by adding D-biotin (final concentration 100 µM), carbenicillin (final concentration 50 mg/L), and chloramphenicol (30 mg/L) to a 5 mL of a MagicMedia E. coli Expression Medium (LIFE TECHNOLOGY INC.), and cultured at 28°C for 28 to 38 hours.

Then, the culture solution was subjected to centrifugation, and thereby Escherichia coli SHuffle T7 pBirAcm was recovered. To the thus-obtained Escherichia coli SHuffle T7 pBirAcm, 2 mL of buffer A2 [0.05 M Tris-HCl (pH 7.4), 0.05 M NaCl, 10% glycerol] was added to obtain a suspension of Escherichia coli SHuffle T7 pBirAcm, and then Escherichia coli SHuffle T7 pBirAcm was disrupted by ultrasonication.

The obtained disrupted cell solution was subjected to a His SpinTrap TALON (GE Healthcare Bioscience), whereby biotinylated TmAFP37-61-KKKK having histidine at the carboxyl-terminus was adsorbed to the column, and then the biotinylated TmAFP37-61-KKKK was eluted from the column using buffer B2 [0.05 M Tris-HCl (pH 8.4), 0.05 M NaCl, 10% glycerol, 0.2 M imidazole].

The biotinylated TmAFP37-61-KKKK thus obtained was checked for purification degree through polyacrylamide electrophoresis, and was found to have a purification degree of 95% or greater. Note that TmAFP37-61-KKK was expressed using a transformant obtained by introducing pET21-TmAFP37-61-KKK-AviTag-C into Escherichia coli SHuffle T7 pBirAcm, TmAFP24-71-KKKK was expressed using a transformant obtained by introducing pET21-TmAFP24-71-KKKK-AviTag-C into Escherichia coli SHuffle T7 pBirAcm, TmAFP37-61-EEEE was expressed using a transformant obtained by introducing pET21-TmAFP37-61-EEEE-AviTag-C into Escherichia coli SHuffle T7 pBirAcm, and TmAFP37-61-HHHH was expressed using a transformant obtained by introducing pET21-TmAFP37-61-HHHH-AviTag-C into Escherichia coli SHuffle T7 pBirAcm. The purification of these proteins was carried out in the same manner as described earlier.

The biotinylated TmAFP37-61-KKK, biotinylated TmAFP24-71-KKKK, biotinylated TmAFP37-61-EEEE, and biotinylated TmAFP37-61-HHHH thus obtained were all found to have a purification degree of 95% or greater.

### [3. Fluorescence labeling of protein]

Fluorescent modification of a protein was carried out by allowing a biotinylated protein to bind to fluorescence-labeled streptavidin through biotin-streptavidin interaction. By this method, it is possible to allow four molecules of biotinylated protein to bind to one molecule of fluorescence-labeled streptavidin, and thus possible to prepare a protein complex that is capable of more strongly binding to a target substance. The following description will discuss a method of fluorescence labeling of biotinylated H-NS60-90. Note that other biotinylated modified TmAFPs were also fluorescence-labeled in a similar manner.

To 60.6 µL of a 50mM Tris-HCl (pH8.4) buffer, 60.6 pmol of fluorescence-labeled streptavidin and 1212 pmol of biotinylated H-NS60-90 were added and mixed, and incubated at room temperature for 1 hour. With this operation, fluorescence-labeled streptavidin and biotinylated H-NS60-90 formed a complex. In this way, fluorescence labeling of H-NS60-90 was carried out. Note that, in this complex, H-NS60-90 is in the form of a tetramer due to the action of streptavidin. That is, in this complex, a tetramer protein, composed of four H-NS60-90 molecules, is labeled with fluorescent dye.

A complex of streptavidin (Biotium) labeled with fluorescent dye CF488A and H-NS60-90 was named "H-NS60-90-Streptavidin-CF488A", and a complex of the above streptavidin and TmAFP37-61-KKKK was named "TmAFP37-61-KKKK-Streptavidin-CF488A".

A complex of streptavidin (invitrogen) labeled with fluorescent dye Cy3 and TmAFP37-61-KKK was named "TmAFP37-61-KKK-Streptavidin-Cy3". A complex of the above streptavidin and TmAFP24-71-KKKK was named "TmAFP24-71-KKKK-Streptavidin-Cy3", a complex of the above streptavidin and TmAFP37-61-EEEE was named "TmAFP37-61-EEEE-Streptavidin-Cy3", and a complex of the above streptavidin and TmAFP37-61-HHHH was named "TmAFP37-61-HHHH-Streptavidin-Cy3".

### [4. Fluorescence microscope observation of amosite with use of fluorescence-labeled protein]

Amosite (JAWE231) serving as a specimen was suspended in buffer C [0.3 M phosphate buffer (pH8.0), 0.3 M NaCl, 0.5% Tween80] so that a concentration of amosite became 1 mg/mL.

A protein labeled with fluorescent dye CF488A (e.g., H-NS60-90-Streptavidin-CF488A, TmAFP37-61-KKKK-Streptavidin-CF488A) was diluted with buffer C so that a concentration of the protein became 100 nM.

A protein labeled with fluorescent dye Cy3 (e.g., TmAFP37-61-KKK-Streptavidin-Cy3, TmAFP24-71-KKKK-Streptavidin-Cy3) was diluted with buffer C so that a concentration of the protein became 40 nM.

The solution containing amosite and the solution containing the fluorescence-labeled protein were mixed at a ratio of 1:1 so as to bind the fluorescence-labeled protein to the amosite.

A mixed solution in which the solution containing amosite and the solution containing the fluorescence-labeled protein were mixed was dripped in an amount of 3 µL to 5 µL onto SLIDE GLASS (white ground edge; thickness of 1 mm), then a cover glass (manufactured by MATSUNAMI, 18 mm × 18 mm) was placed on the mixed solution, and thus a prepared specimen for observation was obtained.

The prepared specimen thus obtained was observed with a phase-contrast/fluorescence microscope. First, observation was carried out in a phase-contrast microscope mode and amosite fiber was confirmed, and then the mode was switched to a fluorescence microscope mode. In the fluorescence microscope mode, a visual field was observed which was identical with a visual field that had been observed in the phase-contrast microscope mode. Note that, for the observation of the fluorescent dye CF488A, a Primo Star iLED (manufactured by Carl Zeiss) was used as the phase-contrast/fluorescence microscope, and a filter set 09 (beam splitter: FT510, excitation filter: BP450-490, fluorescence filter: LP515) was used. For image capture, a microscopic digital camera DP-70 (manufactured by Olympus Corporation) was used. For the observation of the fluorescent dye Cy3, an epifluorescence microscope BX-60 (manufactured by Olympus Corporation) was used as the phase-contrast/fluorescence microscope, and a U-MNG cube (dichroic mirror: DM570, excitation filter: BP530-550, absorption filter: BA590) was used. For image capture, a microscopic digital camera DP-70 (manufactured by Olympus Corporation) was used.

### [5. Fluorescence microscope observation of chrysotile with use of fluorescence-labeled protein]

Chrysotile (JAWE111) serving as a specimen was suspended in buffer C [0.3 M phosphate buffer (pH8.0), 0.3 M NaCl, 0.5% Tween80] so that a concentration of chrysotile became 1 mg/mL.

TmAFP37-61-EEEE labeled with fluorescent dye Cy3 (TmAFP37-61-EEEE-Streptavidin-Cy3) was diluted with buffer C so that a concentration of TmAFP37-61-EEEE-Streptavidin-Cy3 became 15.2 nM.

The solution containing chrysotile and the solution containing the fluorescence-labeled protein were mixed at a ratio of 1:1 so as to bind the fluorescence-labeled protein to the chrysotile.

A mixed solution in which the solution containing chrysotile and the solution containing the fluorescence-labeled protein were mixed was dripped in an amount of 3 µL to 5 µL onto SLIDE GLASS (white ground edge; thickness of 1 mm), then a cover glass (manufactured by MATSUNAMI, 18 mm × 18 mm) was placed on the mixed solution, and thus a prepared specimen for observation was obtained.

The prepared specimen thus obtained was observed with a phase-contrast/fluorescence microscope. First, observation was carried out in a phase-contrast microscope mode and chrysotile fiber was confirmed, and then the mode was switched to a fluorescence microscope mode. In the fluorescence microscope mode, a visual field was observed which was identical with a visual field that had been observed in the phase-contrast microscope mode. For the observation of the fluorescent dye Cy3, an epifluorescence microscope BX-60 (manufactured by Olympus Corporation) was used as the phase-contrast/fluorescence microscope, and a U-MNG cube (dichroic mirror: DM570, excitation filter: BP530-550, absorption filter: BA590) was used. For image capture, a microscopic digital camera DP-70 (manufactured by Olympus Corporation) was used.

### [6. Fluorescence microscope observation of titanium oxide with use of fluorescence-labeled protein]

Titanium oxide (T-8141, manufactured by Sigma-Aldrich) serving as a specimen was suspended in buffer D [0.3 M phosphate buffer (pH7.0), 0.3 M NaCl, 0.5% Tween80] so that a concentration of titanium oxide became 1 mg/mL.

TmAFP37-61-HHHH labeled with fluorescent dye Cy3 (TmAFP37-61-HHHH-Streptavidin-Cy3) was diluted with buffer C so that a concentration of TmAFP37-61-HHHH-Streptavidin-Cy3 became 40 nM.

The solution containing titanium oxide and the solution containing the fluorescence-labeled protein were mixed at a ratio of 1:1 so as to bind the fluorescence-labeled protein to the titanium oxide.

A mixed solution in which the solution containing titanium oxide and the solution containing the fluorescence-labeled protein were mixed was dripped in an amount of 3 µL to 5 µL onto SLIDE GLASS (white ground edge; thickness of 1 mm), then a cover glass (manufactured by MATSUNAMI, 18 mm × 18 mm) was placed on the mixed solution, and thus a prepared specimen for observation was obtained.

The prepared specimen thus obtained was observed with a phase-contrast/fluorescence microscope. First, observation was carried out in a phase-contrast microscope mode and titanium oxide particles were confirmed, and then the mode was switched to a fluorescence microscope mode. In the fluorescence microscope mode, a visual field was observed which was identical with a visual field that had been observed in the phase-contrast microscope mode. For the observation of the fluorescent dye Cy3, an epifluorescence microscope BX-60 (manufactured by Olympus Corporation) was used as the phase-contrast/fluorescence microscope, and a U-MNG cube (dichroic mirror: DM570, excitation filter: BP530-550, absorption filter: BA590) was used. For image capture, a microscopic digital camera DP-70 (manufactured by Olympus Corporation) was used.

### [7. Evaluation of binding specificity]

The following description will discuss a method for evaluating the binding specificity.

As asbestos, amosite (JAWE231) or crocidolite (JAWE331) was used. As non-asbestos fiber, a JFM-standard fibrous sample was used. Specifically, as the JFM-standard fibrous sample, glass wool (GW1), rock wool (RW1), microfiber glass (MG1), refractory (fireproof) fiber (RF1), refractory (fireproof) fiber (RF2), aluminum silicate fiber (RF3), potassium titanate fiber (PT1), silicon carbide whisker (SC1), titanium oxide whisker (TO1), or wollastonite (WO1) was used.

In the evaluation method, a protein (e.g., TmAFP24-71-KKKK) labeled with fluorescence and a test article were mixed in a buffer, and were then observed with a phase-contrast/fluorescence microscope. In a case where fluorescence was detected on the test article, it was determined that "the protein binds to the test article". In a case where fluorescence was not detected on the test article, it was determined that "the protein does not bind to the test article".

Specifically, in a case where amosite (JAWE231), crocidolite (JAWE331), microfiber glass (MG1), refractory (fireproof) fiber (RF1), refractory (fireproof) fiber (RF2), aluminum silicate fiber (RF3), potassium titanate fiber (PT1), silicon carbide whisker (SC1), titanium oxide whisker (TO1), or wollastonite (WO1) was used as the test article, the test article was suspended in buffer C [0.3 M phosphate buffer (pH8.0), 0.3 M NaCl, 0.5% Tween80] so that a concentration of the test article became 1 mg/mL.

In a case where glass wool (GW1) or rock wool (RW1) was used as the test article, the test article was suspended in buffer C so that a concentration of the test article became 5 mg/mL.

TmAFP24-71-KKKK labeled with fluorescent dye Cy3 (TmAFP24-71-KKKK-Streptavidin-Cy3) was diluted with buffer C so that a concentration of TmAFP24-71-KKKK-Streptavidin-Cy3 became 40 nM.

The solution containing the test article and the solution containing the fluorescence-labeled protein were mixed at a ratio of 1:1 so as to bind the fluorescence-labeled protein to the test article.

A mixed solution in which the solution containing the test article and the solution containing the fluorescence-labeled protein were mixed was dripped in an amount of 3 µL to 5 µL onto SLIDE GLASS (white ground edge; thickness of 1 mm), then a cover glass (manufactured by MATSUNAMI, 18 mm × 18 mm) was placed on the mixed solution, and thus a prepared specimen for observation was obtained.

The prepared specimen thus obtained was observed with a phase-contrast/fluorescence microscope. First, observation was carried out in a phase-contrast microscope mode and the test article was confirmed, and then the mode was switched to a fluorescence microscope mode. In the fluorescence microscope mode, a visual field was observed which was identical with a visual field that had been observed in the phase-contrast microscope mode. In a case where fluorescence was detected on the test article, it was determined that "the protein binds to the test article". In a case where fluorescence was not detected on the test article, it was determined that "the protein does not bind to the test article". For the observation of the fluorescent dye Cy3, an epifluorescence microscope BX-60 (manufactured by Olympus Corporation) was used as the phase-contrast/fluorescence microscope, and a U-MNG cube (dichroic mirror: DM570, excitation filter: BP530-550, absorption filter: BA590) was used. For image capture, a microscopic digital camera DP-70 (manufactured by Olympus Corporation) was used.

### [8. Test result]

### [8-1. Test result 1 (Detection of asbestos using substitution product in which threonine in TmAFP is substituted with lysine)]

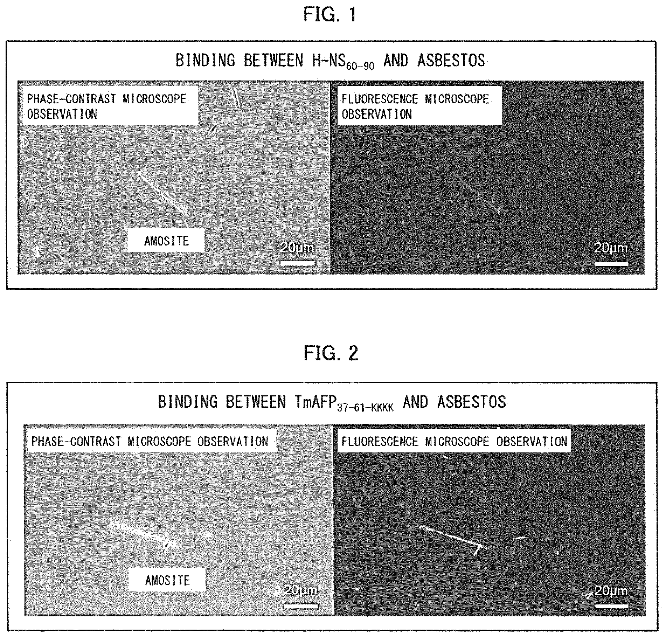

Fig. 1 shows a test result of evaluating binding capacity of a complex of H-NS60-90 labeled with biotin and streptavidin labeled with fluorescent dye CF488A with respect to asbestos (specifically, amosite). As shown in Fig. 1, fluorescence was observed on amosite which is amphibole asbestos and, as in a conventional report, it has been clarified that H-NS60-90 has binding capacity with respect to amosite.

Fig. 2 shows a test result of evaluating binding capacity of a complex of TmAFP37-61-KKKK labeled with biotin and streptavidin labeled with fluorescent dye CF488A with respect to asbestos (specifically, amosite). As shown in Fig. 2, intense fluorescence was observed on amosite which is amphibole asbestos, and it has been clarified that TmAFP37-61-KKKK has strong binding capacity with respect to amosite.

The case in which H-NS60-90 was used was compared with the case in which lysine-substituted TmAFP was used, and it was found that more intense fluorescence was observed on amosite in the case in which the lysine-substituted TmAFP was used. Under the circumstances, fluorescence intensity observed on amosite when H-NS60-90 was used and fluorescence intensity observed on amosite when the lysine-substituted TmAFP was used were evaluated by making a comparison in terms of luminance. Specifically, for images in each of Fig. 1 and Fig. 2, average luminance of amosite was calculated with use of image analysis software ImageJ (Version 1.46v; National Institutes of Health, USA). As a result, average luminance of amosite in Fig. 1 was 30.909, and average luminance of amosite in Fig. 2 was 66.773. That is, it was found that the fluorescence intensity of the present invention was approximately twice (≈ 66.773/30.909) as high as that of the conventional method. The improvement of fluorescence intensity makes it easier to observe asbestos in a test of asbestos which test is carried out under a bright environment (e.g., outdoors). Therefore, the present invention can be considered to be highly useful.

As shown in the above result, a tag having a binding characteristic with respect to an intended substance having a crystal structure was successfully produced by modifying an antifreeze protein which had a highly planar structure adaptable to a surface of ice.

### [8-2. Test result 2 (Detection of asbestos using substitution product in which three threonines in TmAFP are each substituted with lysine)]

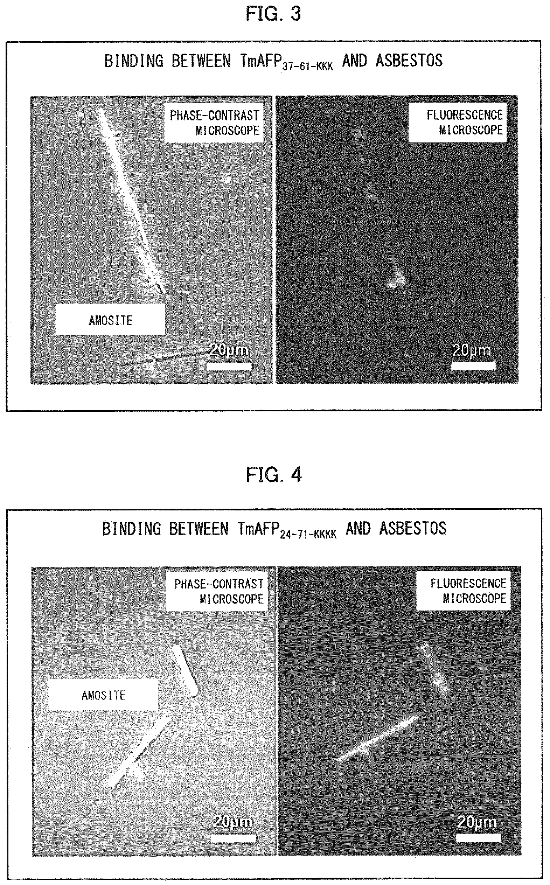

Fig. 3 shows a test result of evaluating binding capacity of a complex of TmAFP37-61-KKK labeled with biotin and streptavidin labeled with fluorescent dye Cy3 with respect to asbestos (specifically, amosite). As shown in Fig. 3, intense fluorescence was observed on amosite which is amphibole asbestos, and it has been clarified that TmAFP37-61-KKK has strong binding capacity with respect to amosite.

That is, it has been clarified that even TmAFP37-61-KKK, in which only three of four threonines that are believed to be involved in binding of an antifreeze protein to an ice crystal are substituted, can bind to asbestos.

### [8-3. Test result 3 (Detection of asbestos using TmAFP24-71-KKKK, and binding specificity)]

Fig. 4 shows a test result of evaluating binding capacity of a complex of TmAFP24-71-KKKK labeled with biotin and streptavidin labeled with fluorescent dye Cy3 with respect to asbestos (specifically, amosite). As shown in Fig. 4, intense fluorescence was observed on amosite which is amphibole asbestos, and it has been clarified that TmAFP24-71-KKKK has strong binding capacity with respect to amosite.

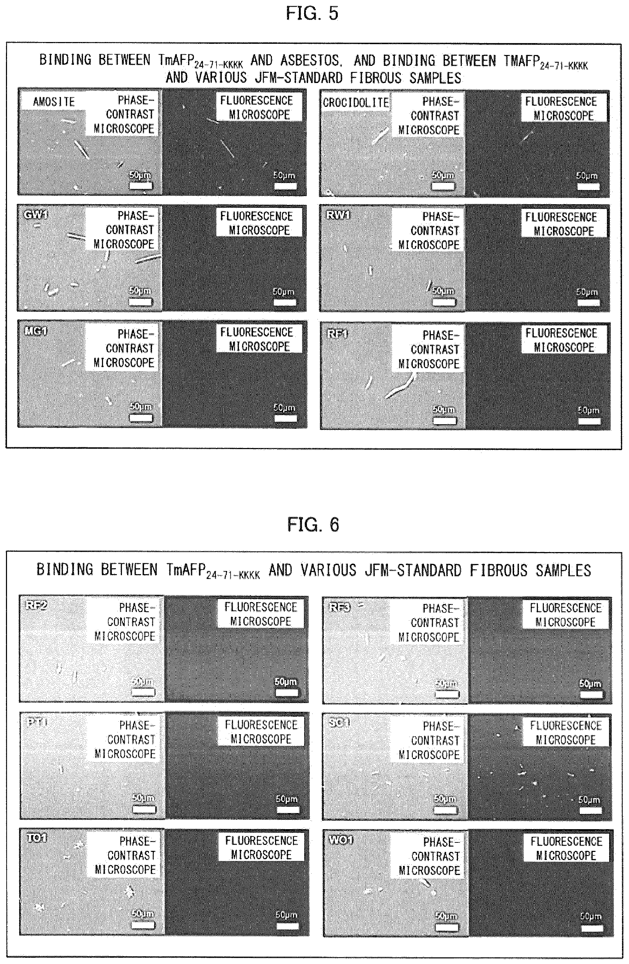

As shown in Fig. 5 and Fig. 6, TmAFP24-71-KKKK bound also to crocidolite which is also amphibole asbestos. Meanwhile, TmAFP24-71-KKKK bound to silicon carbide whisker (SC1) among the JFM-standard fibrous samples which were not asbestos, but did not bind to the other JFM-standard fibrous samples. That is, TmAFP24-71-KKKK seems to highly specifically bind to amphibole asbestos.

### [8-4. Test result 4 (Detection of asbestos using substitution product in which threonine in TmAFP is substituted with glutamic acid)]

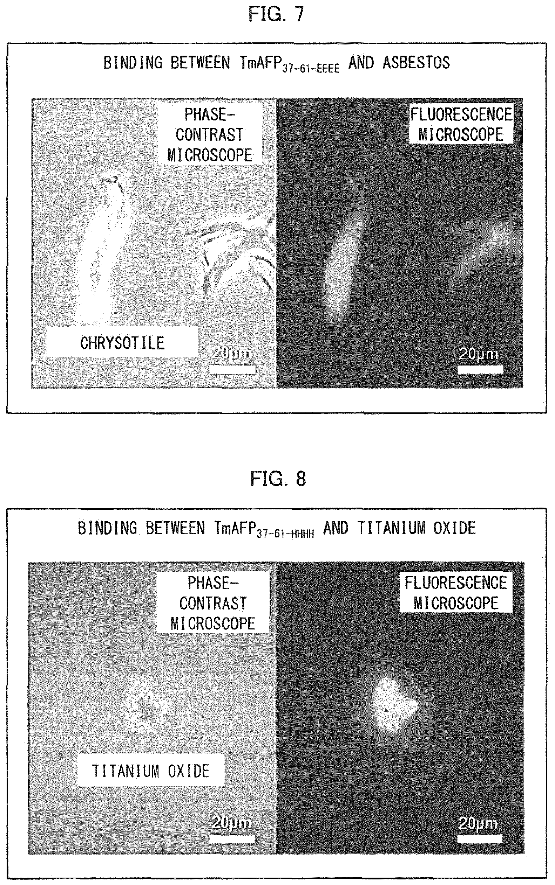

Fig. 7 shows a test result of evaluating binding capacity of a complex of TmAFP37-61-EEEE labeled with biotin and streptavidin labeled with fluorescent dye Cy3 with respect to asbestos (specifically, chrysotile). As shown in Fig. 7, intense fluorescence was observed on chrysotile which is serpentine asbestos, and it has been clarified that TmAFP37-61-EEEE has strong binding capacity with respect to chrysotile.

This represents that a binding target can be changed by changing an amino acid with which threonine is to be substituted.

### [8-5. Test result 5 (Detection of titanium oxide using substitution product in which threonine in TmAFP is substituted with histidine)]

Fig. 8 shows a test result of evaluating binding capacity of a complex of TmAFP37-61-HHHH labeled with biotin and streptavidin labeled with fluorescent dye Cy3 with respect to titanium oxide. As shown in Fig. 8, intense fluorescence was observed on titanium oxide, and it has been clarified that TmAFP37-61-HHHH has strong binding capacity with respect to titanium oxide.

This represents that a binding target can be changed by changing an amino acid by which threonine is to be substituted.

### [9. Preparation of TmAFP phage library, and screening of protein tag (amosite, titanium oxide)]

### [9-1. Preparation of gene coding for antifreeze protein in which random mutations are introduced]

Preparation of a gene, which codes for a protein having the same amino acid sequence as an ice-binding domain derived from an antifreeze protein except that random amino acid mutations are introduced, was carried out.

As the antifreeze protein, Tenebiro molitor antifreeze protein (TmAFP, Database: GenBank, Accession number: AAB70750) was used.

In regard to the amino acid region (SEQ ID NO:2) consisting of the amino acids at the 37th to 61st positions from the amino-terminus of TmAFP, threonines (T) at the 39th, 41st, 51st, and 53rd positions from the amino-terminus of TmAFP are believed to be involved in binding to an ice crystal. In view of this, a mutant TmAFP gene, which codes for a mutant protein in which the four threonines (T) are substituted with respective random amino acid mutations, was designed.

Specifically, a mutant TmAFP gene, in which codons coding for the respective four threonines (T) are substituted with an NNK codon set (N = A, T, G, C; K = G, T) which is degenerate codons, was prepared. PCR was carried out using a nucleotide represented by SEQ ID NO:21 as a template and using oligonucleotide primer L1 and oligonucleotide primer L2 as primers, and thus a mutant TmAFP gene in which random mutations are introduced was prepared. Note that the PCR was carried out with use of a PrimeSTAR (registered trademark) Max DNA Polymerase (manufactured by Takara Bio) in accordance with the protocol of Takara Bio:
L1: 5'-GCCCAGCCGGCCATGGCAAAT-3' (SEQ ID NO:22),
L2: 5'-CGCACCTGCGGCCGCACTACCACCACCTGAACCACCAC-3' (SEQ ID NO:23).

### [9-2. Preparation of phage library displaying TmAFP in which random mutations are introduced]

The mutant TmAFP gene which is described in the above [9-1] and in which the random mutations are introduced was inserted, by In-Fusion cloning, into a region between the restricted enzyme sites Sifl and Notl of a phagemid vector pCANTAB5E2, and Escherichia coli TG1 was transformed with use of the phagemid vector. Further, the Escherichia coli, which had been transformed, was infected with a helper phage, and thus a library of phages displaying mutant TmAFP proteins in which random mutations are introduced was prepared. Details are described below.

First, the mutant TmAFP gene in which random mutations are introduced was inserted into pCANTAB5E2. in the In-Fusion cloning, a gene of interest was inserted into a linearized vector by an In-Fusion reaction of the linearized vector with the gene of interest.

Specifically, a linearized vector was prepared by carrying out inverse PCR with use of pCANTAB5E2 as a template and with use of oligonucleotide primer L3 and oligonucleotide primer L4 as primers. The mutant TmAFP gene, which was prepared in the above [9-1] and in which the random mutations were introduced, was inserted into the linearized pCAN-TAB5E2 by In-Fusion cloning:
L3: 5'-GCGGCCGCAGGTGCGCCGGTGCCG-3' (SEQ ID NO:24),
L4: 5'-CATGGCCGGCTGGGCCGCATAGAAAG-3' (SEQ ID NO:25).

The In-Fusion cloning was carried out with use of an In-Fusion HD Cloning Kit (manufactured by Takara Bio) in accordance with the protocol of Takara Bio. Escherichia coli TG1 was transformed by electroporation (1700 V, 200 Ω, 25 µF) with use of the thus-obtained phagemid vector pCANTAB5E2 into which the mutant TmAFP gene, in which random mutations were introduced, was inserted.

Next, the Escherichia coli TG1, which had been transformed, was infected with a helper phage, and thus a phage library was prepared.

Specifically, the Escherichia coli TG1, which had been transformed, was subjected to shake culture under a condition of 37°C for one hour in 10 mL of a 2×YT (2×YTG) liquid medium which contained glucose at a concentration of 2%. Carbenicillin (final concentration 100 µg/mL) and 4×1010 pfu of an m13KO7 helper phage (manufactured by New England Biolabs) were added to a culture solution, and then the culture solution was subjected to shake under a condition of 37°C for one hour. Subsequently, the culture solution was subjected to centrifugation (1000 g, 4°C, for 10 minutes), then a supernatant was removed from the culture solution, and thus superfluous helper phages were eliminated.

A precipitate (Escherichia coli) which had been obtained by the centrifugation was suspended again in 10 mL of a 2×YT liquid medium, then carbenicillin (final concentration 100 µg/mL) and kanamycin (final concentration 50 µg/mL) were added to a suspended matter thus obtained, and then Escherichia coli was cultured under a condition of 37°C for 17 hours.

After that, a cultured matter thus obtained was subjected to centrifugation (1000 g, 4°C, 20 minutes) so as to precipitate Escherichia coli, and 10 mL of a supernatant (phage fraction) was recovered. Then, 2 mL of 20% polyethylene glycol containing 2.5 M of sodium chloride was added and suspended in the supernatant (10 mL), and phages were precipitated by leaving a suspended matter thus obtained still in ice for one hour.

After that, a supernatant was removed by centrifugation (10000 g, 4°C, for 20 minutes), then a precipitate (phage) was suspended in 3 mL of a PBS, and thus phages were purified. In order to further purify the phages, the suspension was subjected to centrifugation (11600 g, 4°C, for three minutes) twice, and then 2.5 mL of a supernatant (phage fraction) was recovered. The supernatant was mixed with 2.5 mL of 80% glycerol, and thus a phage library solution was prepared. The phage library solution was preserved under a condition of -20°C until being used.

### <Test result>

A phage library size was calculated and was found to be 1.22×106. The threonines (T) at the 39th, 41st, 51st, and 53rd positions from the amino-terminus are substituted with the NNK codon set (N = A, T, G, C; K = G, T) which is degenerate codons, and therefore a possible combination of mutations is (4 × 4 × 2)4 = 1.048×106. From this, the size of the phage library prepared this time seems to cover all possible combinations of mutations. Moreover, TmAFP genes which had been randomly selected from the phage library constructed this time was subjected to sequence analysis, and it was consequently confirmed that all the TmAFP genes had respective different sequences.

### [9-3. Obtainment of mutant TmAFP that binds to asbestos (amosite) or titanium oxide by biopanning]

With use of the phage library prepared in the above [9-2] and containing phages displaying the TmAFP proteins in which random mutations are introduced, screening of a mutant TmAFP, which binds to asbestos (amosite) or titanium oxide, was carried out. Amosite (JAWE231) was used as asbestos, and titanium oxide (T-8141, manufactured by Sigma-Aldrich) was used as titanium oxide.

Specifically, each biopanning was carried out by the following operating method. First, 1 mg of amosite particles or 10 mg of titanium oxide particles were suspended in 2 mL of buffer A [Tris-HCl (pH9.0), 0.3 M NaCl, 0.5% Tween80 (registered trademark)] in a microtube having a capacity of 2 mL. A suspension thus obtained was subjected to centrifugation (11600 g, 4°C, for three minutes), and thus the amosite particles or the titanium oxide particles were precipitated.

To a precipitate thus obtained, a diluted solution (obtained by diluting, with buffer A, the phage library solution prepared in the above [9-2]) of the phage library which had been adjusted to have 5.0×109 particles was added so as to suspend the precipitate in the diluted solution, and a suspension thus obtained was stirred for 30 minutes. Subsequently, the suspension was subjected to centrifugation (11600 g, 4°C, for three minutes), then a supernatant was removed, and thus unbound phages were removed.

To a precipitate which had been obtained by the centrifugation, 500 µL of buffer A was added so as to suspend the precipitate in buffer A, and an entire suspension thus obtained was transferred to a new microtube having a capacity of 2 mL. Subsequently, the suspension was subjected to centrifugation (11600 g, 4°C, for three minutes), then a supernatant was removed, and 500 µL of buffer A was added to the precipitate, and the precipitate was washed. This washing operation was repeated five times, and thus phages which did not have affinity to the amosite particles or to the titanium oxide particles were removed.

In the fifth washing operation, buffer A was added to a precipitate, which had been obtained by the centrifugation, so as to suspend the precipitate in buffer A, and an entire suspension thus obtained was transferred to a new microtube having a capacity of 1.5 mL. Subsequently, the suspension was subjected to centrifugation (11600 g, 4°C, for three minutes), then a supernatant was removed, and the precipitate was suspended in 500 µL of a 2×YT liquid medium. Then, 490 µL of the 2×YT liquid medium was mixed with 10 mL of a culture solution of Escherichia coli TG1 (cultured up to a logarithmic growth phase in a 2×YTG medium), and a mixture thus obtained was subjected to shake at 37°C for one hour. From this, Escherichia coli TG1 was infected with the phage.

Carbenicillin (final concentration 100 µg/mL) and 4×1010 pfu of an m13KO7 helper phage (manufactured by New England Biolabs) were added to a culture solution, and then the culture solution was subjected to shake at 37°C for one hour. Subsequently, the culture solution was subjected to centrifugation (1000 g, 4°C, for 10 minutes), then a supernatant was removed, and thus superfluous helper phages were eliminated.

A precipitate which had been obtained by the centrifugation was suspended again in 10 mL of a 2×YT liquid medium, then carbenicillin (final concentration 100 µg/mL) and kanamycin (final concentration 50 µg/mL) were added to a suspension thus obtained, and then the suspension was cultured under a condition of 37°C for 17 hours. After that, a cultured matter thus obtained was subjected to centrifugation (1000 g, 4°C, 20 minutes) so as to precipitate Escherichia coli, and 10 mL of a supernatant (phage fraction) was recovered. Then, 2 mL of 20% polyethylene glycol containing 2.5 M of sodium chloride was added and suspended in the supernatant (10 mL), and phages were precipitated by leaving a suspension thus obtained still in ice for one hour. After that, the suspension was subjected to centrifugation (10000 g, 4°C, for 20 minutes), then a supernatant was removed, and then precipitated phages were suspended in 1 mL of a PBS. As such, phages were purified.

In order to further purify the phages, the suspension of phages was subjected to centrifugation (11600 g, 4°C, for three minutes) twice, and then 950 µL of a supernatant (phage fraction) was recovered. The supernatant was mixed with 950 µL of 80% glycerol. A mixture thus obtained was preserved at -20°C until being used in next biopanning. The above operations constitute first biopanning. Then, second and subsequent biopannings were carried out in a similar manner. Ultimately, the amosite and the titanium oxide were each subjected to biopanning six times in total.

A base sequence of a mutant TmAFP gene, which is for a mutant TmAFP displayed by a phage obtained after the sixth panning operation and bound to the titanium oxide or the amosite, was determined.

First, PCR was carried out with use of oligonucleotide primer L5 and oligonucleotide primer L6 (which are described later), and thus a TmAFP gene was amplified. The PCR was carried out with use of a PrimeSTAR (registered trademark) Max DNA Polymerase (manufactured by Takara Bio) in accordance with the protocol of Takara Bio. An amplified product of the TmAFP gene which had been amplified by the PCR was separated by electrophoresis with use of a 2% agarose gel, and the length of the amplified product was confirmed. Then, the amplified product was purified from the agarose gel. A base sequence of the TmAFP gene was determined using the purified amplified product as a template, by a dideoxy termination method with use of oligonucleotide primer L6 (using a CEQ DTCS Quick start kit, manufactured by Beckman Coulter). Migration and data analysis of the amplified product were carried out with use of a capillary sequencer (CEQ8000, manufactured by Beckman Coulter):
L5: 5'-CCATGATTACGAATTCTGGAGCC-3' (SEQ ID NO:26),
L6: 5'-CGATCTAAAGTTTTGTCGTCTTTCC-3' (SEQ ID NO:27).

### <Test result>

By sequence analysis, an amino acid sequence of a mutant TmAFP protein, which is displayed by a phage obtained after the sixth panning operation and bound to the amosite or the titanium oxide, was determined.

Table 2 below shows information on amino acid sequences of mutant TmAFP proteins which bind to amosite (in particular, information on amino acids at the 39th, 41st, 51st, and 53rd positions from the amino-terminus), and Table 3 below shows information on amino acid sequences of mutant TmAFP proteins which bind to titanium oxide (in particular, information on amino acids at the 39th, 41st, 51st, and 53rd positions from the amino-terminus).

**Table 2**

| | Position form amino-terminus | | | | Frequency of occurrence (/19) |
|---|---|---|---|---|---|
| | 39 | 41 | 51 | 53 | |
| Wild type | T | T | T | T | - |
| 1 | A | K | V | C | 14 |
| 2 | N | K | R | K | 2 |
| 3 | K | V | K | K | 1 |
| 4 | K | K | E | K | 1 |
| 5 | K | H | K | R | 1 |

**Table 3**

| | Position form amino-terminus | | | | Frequency of occurrence (/21) |
|---|---|---|---|---|---|
| | 39 | 41 | 51 | 53 | |
| Wild type | T | T | T | T | - |
| 1 | I | K | R | K | 10 |
| 2 | R | P | K | K | 3 |
| 3 | K | V | K | K | 2 |
| 4 | S | G | K | K | 1 |
| 5 | S | K | K | R | 1 |
| 6 | N | K | R | K | 1 |
| 7 | S | K | Y | K | 1 |
| 8 | K | K | T | R | 1 |
| 9 | F | K | R | K | 1 |

From the above results, it has been clarified that the present invention makes it possible to produce a protein tag that is suitable for each of binding-target substances (e.g., asbestos, titanium oxide, and the like).

The present invention can be used in, for example, detection of a substance having a crystal structure.

## Claims

1. A protein tag which binds to a substance having a crystal structure, the protein tag comprising:
- a binding domain whose amino acid sequence is a mutated version of an amino acid sequence of an ice-binding domain derived from an antifreeze protein.

2. The protein tag according to claim 1, wherein mutation in the mutated version is amino acid substitution.

3. The protein tag according to claim 2, wherein the mutation is such that threonine in the ice-binding domain of a wild type is substituted with an amino acid "a" different from threonine.

4. The protein tag according to claim 3, wherein:
- the ice-binding domain of a wild type contains a motif represented by the following amino acids; and
- the threonine substituted is at least one selected from the group consisting of threonine at the 1st position from the amino-terminus of the motif and threonine at the 3rd position from the amino-terminus of the motif:
Motif: -TCTXSXXCXXAX-
(where T represents threonine, C represents cysteine, S represents serine, A represents alanine, and X represents any amino acid).

5. The protein tag according to claim 3 or 4, wherein
- in a case where the ice-binding domain of a wild type contains an amino acid of the same kind as the amino acid "a",
- the amino acid, which is originally contained, of the same kind as the amino acid "a" is substituted with an amino acid "b" different from the amino acid "a".

6. A method for screening a protein tag which binds to a target substance having a crystal structure, the method comprising the steps of:
- bringing a phage and a target substance having a crystal structure into contact with each other, the phage displaying, by a phage display method, a protein tag that includes a binding domain whose amino acid sequence is a mutated version of an amino acid sequence of an ice-binding domain derived from an antifreeze protein; and
- recovering the phage bound to the target substance having a crystal structure.

7. A kit for screening a protein tag which binds to a target substance having a crystal structure, the kit comprising a phage vector or a phagemid vector each of which contains a gene coding for a protein tag, the protein tag including a binding domain whose amino acid sequence is a mutated version of an amino acid sequence of an ice-binding domain derived from an antifreeze protein.

8. A method for producing a protein tag which binds to a target substance having a crystal structure, the method comprising the steps of:
- bringing a phage and a target substance having a crystal structure into contact with each other, the phage displaying, by a phage display method, a protein tag that includes a binding domain whose amino acid sequence is a mutated version of an amino acid sequence of an ice-binding domain derived from an antifreeze protein; and
- recovering the phage bound to the target substance having a crystal structure.


SEQUENCE LISTING

<110>  Hiroshima University

<120>  Protein tag binding to substance with crystal structure and use of
the protein tag

<130>  HU16196

<150>  JP 2015-185940
<151>  2015-09-18

<160>  27

<170>  PatentIn version 3.5

<210>  1
<211>  12
<212>  PRT
<213>  Artificial Sequence

<220>
<223>  Artificially Synthesized Sequence



<220>
<221>  misc_feature
<222>  (4)..(4)
<223>  Xaa can be any naturally occurring amino acid

<220>
<221>  misc_feature
<222>  (6)..(7)
<223>  Xaa can be any naturally occurring amino acid

<220>
<221>  misc_feature
<222>  (9)..(10)
<223>  Xaa can be any naturally occurring amino acid

<220>
<221>  misc_feature
<222>  (12)..(12)
<223>  Xaa can be any naturally occurring amino acid

<400>  1

Thr Cys Thr Xaa Ser Xaa Xaa Cys Xaa Xaa Ala Xaa
1               5                   10


<210>  2
<211>  25
<212>  PRT
<213>  Artificial Sequence

<220>
<223>  Artificially Synthesized Sequence

<400>  2

Ala Asn Thr Cys Thr Gly Ser Thr Asp Cys Asn Thr Ala Gln Thr Cys
1               5                   10                  15

Thr Asn Ser Lys Asp Cys Phe Glu Ala
            20                  25

<210>  3
<211>  48
<212>  PRT
<213>  Artificial Sequence

<220>
<223>  Artificially Synthesized Sequence

<400>  3

Asn Ala Val Thr Cys Thr Asn Ser Gln His Cys Val Lys Ala Asn Thr
1               5                   10                  15

Cys Thr Gly Ser Thr Asp Cys Asn Thr Ala Gln Thr Cys Thr Asn Ser
            20                  25                  30

Lys Asp Cys Phe Glu Ala Asn Thr Cys Thr Asp Ser Thr Asn Cys Tyr
        35                  40                  45

<210>  4
<211>  339
<212>  DNA
<213>  Artificial Sequence

<220>
<223>  Artificially Synthesized Sequence

<400>  4
atggcattca aaacgtgtgg tttttcaaaa aaatggttag taatagcagt tatagttatg      60

tgtttgtgta ccgagtgtta ttgccaatgc actgggggtg ctgattgcac tagttgtaca     120

ggagcatgca ctggttgtgg aaactgtcca aatgcagtaa cgtgtaccaa ttctcaacat     180

tgtgtcaagg caaatacatg tactgggtct acagattgta atacagccca gacgtgtaca     240

aactcaaaag actgttttga agccaacaca tgtactgact caaccaactg ttacaaagct     300

acagcctgta ccaattcatc aggatgtccc ggacattaa                            339

<210>  5
<211>  112
<212>  PRT
<213>  Artificial Sequence

<220>
<223>  Artificially Synthesized Sequence

<400>  5

Met Ala Phe Lys Thr Cys Gly Phe Ser Lys Lys Trp Leu Val Ile Ala
1               5                   10                  15

Val Ile Val Met Cys Leu Cys Thr Glu Cys Tyr Cys Gln Cys Thr Gly
                20                  25                  30

Gly Ala Asp Cys Thr Ser Cys Thr Gly Ala Cys Thr Gly Cys Gly Asn
        35                  40                  45

Cys Pro Asn Ala Val Thr Cys Thr Asn Ser Gln His Cys Val Lys Ala
    50                  55                  60

Asn Thr Cys Thr Gly Ser Thr Asp Cys Asn Thr Ala Gln Thr Cys Thr
65                  70                  75                  80

Asn Ser Lys Asp Cys Phe Glu Ala Asn Thr Cys Thr Asp Ser Thr Asn
                85                  90                  95

Cys Tyr Lys Ala Thr Ala Cys Thr Asn Ser Ser Gly Cys Pro Gly His
            100                 105                 110


<210>  6
<211>  25
<212>  PRT
<213>  Artificial Sequence

<220>
<223>  Artificially Synthesized Sequence

<400>  6

Ala Asn Lys Cys Lys Gly Ser Thr Asp Cys Asn Thr Ala Gln Lys Cys
1               5                   10                  15

Lys Asn Ser Lys Asp Cys Phe Glu Ala
            20                  25


<210>  7
<211>  25
<212>  PRT
<213>  Artificial Sequence

<220>
<223>  Artificially Synthesized Sequence

<400>  7

Ala Asn Lys Cys Lys Gly Ser Thr Asp Cys Asn Thr Ala Gln Lys Cys
1               5                   10                  15

Thr Asn Ser Lys Asp Cys Phe Glu Ala
            20                  25


<210>  8
<211>  25

<212>  PRT
<213>  Artificial Sequence

<220>
<223>  Artificially Synthesized Sequence

<400>  8

Ala Asn Glu Cys Glu Gly Ser Thr Asp Cys Asn Thr Ala Gln Glu Cys
1               5                   10                  15

Glu Asn Ser Lys Asp Cys Phe Glu Ala
            20                  25

<210>  9
<211>  25
<212>  PRT
<213>  Artificial Sequence

<220>
<223>  Artificially Synthesized Sequence

<400>  9

Ala Asn His Cys His Gly Ser Thr Asp Cys Asn Thr Ala Gln His Cys
1               5                   10                  15

His Asn Ser Lys Asp Cys Phe Glu Ala
            20                  25

<210>  10
<211>  48
<212>  PRT
<213>  Artificial Sequence

<220>
<223>  Artificially Synthesized Sequence

<400>  10

Asn Ala Val Thr Cys Thr Asn Ser Gln His Cys Val Gly Ala Asn Lys
1               5                   10                  15

Cys Lys Gly Ser Thr Asp Cys Asn Thr Ala Gln Lys Cys Lys Asn Ser
            20                  25                  30

Gly Asp Cys Phe Glu Ala Asn Thr Cys Thr Asp Ser Thr Asn Cys Tyr
        35                  40                  45

<210>  11
<211>  49
<212>  DNA
<213>  Artificial Sequence

<220>
<223>  Artificially Synthesized Sequence

<400>  11
gctcagaaaa tcgaatggca cgaacaccac caccaccacc actgaacta                49


<210>  12
<211>  51
<212>  DNA
<213>  Artificial Sequence

<220>
<223>  Artificially Synthesized Sequence

<400>  12
ctcgaagatg tcgttcagac cgccaccctc gagtgcggcc gcaagcttgt c             51


<210>  13
<211>  27
<212>  DNA
<213>  Artificial Sequence

<220>
<223>  Artificially Synthesized Sequence

<400>  13
catatgcaat atcgcgaaat gctgatc                                        27


<210>  14
<211>  29
<212>  DNA
<213>  Artificial Sequence

<220>
<223>  Artificially Synthesized Sequence

<400>  14
ggatccaaac aacgtttagc tttggtgcc                                      29


<210>  15
<211>  64
<212>  DNA
<213>  Artificial Sequence

<220>
<223>  Artificially Synthesized Sequence

<400>  15
aatagcaaag attgttttga agccggtggt ggtggtagcg gtggcggtct gaacgacatc    60

ttcg                                                                 64


<210>  16
<211>  75
<212>  DNA
<213>  Artificial Sequence

<220>
<223>  Artificially Synthesized Sequence

<400>  16
tttacatttc tgtgcggtat tacaatcggt gctaccttta catttatttg ccatatgtat      60

atctccttct taaag                                                        75


<210>  17
<211>  75
<212>  DNA
<213>  Artificial Sequence

<220>
<223>  Artificially Synthesized Sequence

<400>  17
ggtacatttc tgtgcggtat tacaatcggt gctaccttta catttatttg ccatatgtat      60

atctccttct taaag                                                        75


<210>  18
<211>  75
<212>  DNA
<213>  Artificial Sequence

<220>
<223>  Artificially Synthesized Sequence

<400>  18
ttcacattcc tgtgcggtat tacaatcggt gctaccttca cattcatttg ccatatgtat      60

atctccttct taaag                                                        75


<210>  19
<211>  75
<212>  DNA
<213>  Artificial Sequence

<220>
<223>  Artificially Synthesized Sequence

<400>  19
gtgacagtgc tgtgcggtat tacaatcggt gctaccgtga cagtgatttg ccatatgtat      60

atctccttct taaag                                                        75


<210>  20
<211>  177
<212>  DNA
<213>  Artificial Sequence

<220>
<223>  Artificially Synthesized Sequence

<400>  20
ggaattccat atgaatgcag tgacctgtac caatagccag cattgtgttg gtgccaataa      60

atgtaaaggt agcaccgatt gtaataccgc acagaaatgt aaaaatagcg gtgattgctt     120

tgaagccaat acctgcaccg atagcaccaa ttgttatggt agtgcggccg caagagc        177

<210>  21
<211>  118
<212>  DNA
<213>  Artificial Sequence

<220>
<223>  Artificially Synthesized Sequence



<220>
<221>  misc_feature
<222>  (23)..(24)
<223>  n is a, c, g, or t

<220>
<221>  misc_feature
<222>  (25)..(25)
<223>  k is g, or t

<220>
<221>  misc_feature
<222>  (29)..(30)
<223>  n is a, c, g, or t

<220>
<221>  misc_feature
<222>  (31)..(31)
<223>  k is g, or t

<220>
<221>  misc_feature
<222>  (59)..(60)
<223>  n is a, c, g, or t

<220>
<221>  misc_feature
<222>  (61)..(61)
<223>  k is g, or t

<220>
<221>  misc_feature
<222>  (65)..(66)
<223>  n is a, c, g, or t

<220>
<221>  misc_feature
<222>  (67)..(67)
<223>  k is g, or t

<400>  21
ggcccagccg gccatggcaa atnnktgtnn kggtagcacc gattgtaata ccgcacagnn      60

ktgtnnkaat agcaaagatt gttttgaagc cggtggtggt ggttcaggtg gtggtagt       118


<210>  22
<211>  21
<212>  DNA
<213>  Artificial Sequence

<220>
<223>  Artificially Synthesized Sequence

<400>  22
gcccagccgg ccatggcaaa t                                                21


<210>  23
<211>  38
<212>  DNA
<213>  Artificial Sequence

<220>
<223>  Artificially Synthesized Sequence

<400>  23
cgcacctgcg gccgcactac caccacctga accaccac                              38


<210>  24
<211>  24
<212>  DNA
<213>  Artificial Sequence

<220>
<223>  Artificially Synthesized Sequence

<400>  24
gcggccgcag gtgcgccggt gccg                                             24


<210>  25
<211>  26
<212>  DNA
<213>  Artificial Sequence

<220>
<223>  Artificially Synthesized Sequence

<400>  25
catggccggc tgggccgcat agaaag                                           26


<210>  26
<211>  23
<212>  DNA
<213>  Artificial Sequence

<220>
<223>  Artificially Synthesized Sequence

<400>  26
ccatgattac gaattctgga gcc                                              23


<210>  27
<211>  25
<212>  DNA
<213>  Artificial Sequence

<220>
<223>  Artificially Synthesized Sequence

<400>  27
cgatctaaag ttttgtcgtc tttcc                                            25